# EUROPEAN PATENT SPECIFICATION

(11) **EP 1 285 579 B1**
(45) Date of publication and mention of the grant of the patent: **02.11.2005**
(21) Application number: 02255443.0
(22) Date of filing: 05.08.2002
(51) Int. Cl.: A01N 47/44

(54) **Bactericidal guanidine derivatives, dermally applicable composition, washing composition, and antibacterial fibre aggregate**
Bakterizide Guanidin-Derivate, dermal applizierbare Zusammensetzungen, Zusammensetzungen zum Waschen und antibakterielle Faseraggregate
Dérivés bactéricides de guanidine, composition applicable sur la peau, compositions de lavage et agrégats antibactériens de fibres

(30) Priority: 21.08.2001 JP 2001250557
(43) Date of publication of application: 26.02.2003
(73) Proprietor: Ajinomoto Co., Inc., Tokyo 104-8315 (JP)
(72) Inventor: Yumioka, Ryosuke, C/o Ajinomoto Co., Inc., Kawasaki-shi, Kanagawa 210-0801 (JP); Nakanishi, Noriyuki, C/o Ajinomoto Co., Inc., Kawasaki-shi, Kanagawa 210-0801 (JP); Yokota, Hirofumi, C/o Ajinomoto Co., Inc., Kawasaki-shi, Kanagawa 210-0801 (JP)
(74) Representative: Nicholls, Kathryn Margaret

(56) References cited:
- WO-A-01/21210
- DE-A- 2 131 404
- US-A- 4 371 577
- US-A- 4 762 949
- K.HONMA ET AL.: "Aplysillamides A and B, New Antimicrobial Gunaidine Alkaloides from the Okinawan Marine Sponge Psammaplysilla purea" TETRAHEDRON., vol. 51, no. 13, 27 March 1995 (1995-03-27), pages 3745-3748, XP002222329 ELSEVIER SCIENCE PUBLISHERS, AMSTERDAM., NL ISSN: 0040-4020
- DATABASE WPI Section Ch, Week 197716 Derwent Publications Ltd., London, GB; Class D22, AN 1977-28413Y XP002222330 & JP 52 010458 B (AJINOMOTO KK), 24 March 1977 (1977-03-24)
- DATABASE WPI Section Ch, Week 199639 Derwent Publications Ltd., London, GB; Class D22, AN 1996-387163 XP002222331 & JP 08 183705 A (AJINOMOTO KK), 16 July 1996 (1996-07-16)
- DATABASE WPI Section Ch, Week 199506 Derwent Publications Ltd., London, GB; Class D21, AN 1995-041143 XP002222332 & JP 06 321727 A (LION CORP), 22 November 1994 (1994-11-22)

## Description

The present invention relates to an antiseptic or bactericide employing an amide group-containing guanidine derivative or its salt. Moreover, the present invention relates to a dermally applicable composition, washing composition, antibacterial fiber aggregate, method for eradicating a microorganism and method for inhibiting the proliferation of a microorganism, all employing the antiseptic or bactericide.

Conventional products in water-containing dosage form including cosmetic products or skin external formulations, such as lotions, emulsions, ointments, face lotions, milky lotions and creams, wet tissues, oral compositions and the like, employ various antiseptics or bactericides for the purpose of preventing any denaturation due to contamination with microorganisms such as bacteria and fungi during production and use, and also for the purpose of eradicating bacteria and microorganisms existing in the oral cavity, on the skin or hairs.

Such antiseptics and bactericides employed in the products described above may for example be phenols such as isopropylmethylphenol, p-oxybenzoates, phenoxyethanol, hinokithiol and the like, acids such as benzoic acid and its salts, salicylic acid and its salts, dehydroacetic acid and its salts, sorbic acid and its salts and the like, quaternary ammoniums such as benzalkonium chloride, benzethonium chloride, alkyltrimethylammonium chlorides and the like, amphoteric surfactants such as alkylaminoethylglycine hydrochloride, stearylhydroxyethylbetaine sodium chloride and the like, photosensitive elements, zincundecylenoate, chlorhexidine gluconate (Hibiten), iodine and the like.

However, any of the antiseptics and bactericides listed above is limited under the Japanese Pharmacopoeia, Standards of Cosmetic Ingredients and Standards of Food Additives, with regard to the quantity to be incorporated, and it is often that an amount capable of exerting an actually effective antibacterial activity cannot be incorporated.

On the other hand, recent interest by society in the safety and an increasing naturalness-oriented trend are encouraging studies on various amino acid derivatives. N-long chain acyl basic amino acid derivatives or acid addition salts thereof, which are naturally occurring amino acid derivatives and are cationic active agents similarly like the quaternary ammonium salts, have been known for a long time as bactericidal washing agents (Japanese Patent Application Publication 51-5413), and one of such substances, namely, N-cocoyl-L-arginine ethyl ester DL-pyrrolidone carboxylate, is marketed under the trade name of "CAE", and exhibits an excellent safety and satisfactory antiseptic or bactericidal activity.

Nevertheless, since an N-long chain acyl basic amino acid derivative is usually employed as a salt and is water soluble, a salt of the acid derivative may sometimes be hydrolyzed at an ester group in its molecule.

On the other hand, an amino group-containing guanidine derivative or a salt thereof having a structure formed by deleting an ester group as a hydrolysis site from an N-long chain acyl basic amino acid derivative is employed as an excellent surfactant when compared with the commonly used quaternary ammonium salt, since it exhibits excellent adsorption performance on hair and imparts the hair with a pliability, moisturizing effect and satisfactory finish, as described in Japanese Patent Application Laid-Open 2-243614, Japanese Patent Application Laid-Open 4-49221 and Japanese Patent Application Laid-Open 4-49222. Also as disclosed in Japanese Patent Application Laid-Open 6-321727, an amino group-containing guanidine derivative or a salt thereof is known to exhibit an excellent moisturizing effect, satisfactory spreading on the skin and smooth touch without any stickiness, when utilized in a skin external formulation.

Nevertheless, an amino group-containing guanidine derivative or a salt thereof is known to be utilizable as a texture modifier in a hair cosmetic material and skin external formulation, but it has not been utilized or examined as an antiseptic or bactericide and actually there are no known examples of the utilization of the amino group-containing guanidine derivative or a salt in a wet tissue product or oral formulation.

In this description, 'bactericide' means an agent that eradicates microorganisms and 'antiseptic' means an agent that inhibits proliferation of microorganisms.

It is an object of the present invention to provide an antiseptic and/or bactericide having an excellent antibacterial effect together with a high chemical stability. It is also an object of this invention to provide a dermally applicable composition, washing composition, antibacterial fiber aggregate, method for eradicating a microorganism and method for inhibiting the proliferation of a microorganism, all employing the antiseptic and/or bactericide. For the purpose of accomplishing the objective described above, the inventors made an effort and finally discovered that a certain amide group-containing guanidine derivative or its salt has an excellent antibacterial activity together with a high chemical stability, thus establishing the present invention.

The antiseptic or bactericide according to one aspect of the present invention comprises at least an amide group-containing guanidine derivative represented by General Formula (I) or a salt thereof. 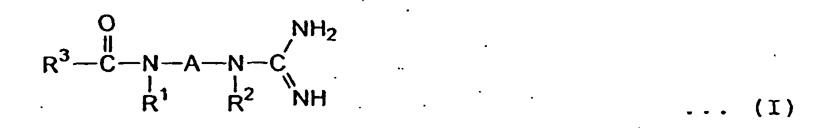 wherein R¹ and R² are the same or different and each denotes a hydrogen atom, a straight or branched alkyl group or alkenyl group having 1 to 4 carbon atoms, R³ denotes a straight or branched alkyl group or alkenyl group having 11 to 13 carbon atoms, and A denotes a straight or branched alkylene group or alkenylene group having 1 to 10 carbon atoms.

Thedermally applicable composition according to another aspect of the present invention comprises at least one of the bactericide and the antiseptic according to the first aspect of the present invention.

The washing composition according to still another aspect of the present invention comprises at least one of the bactericide and the antiseptic according to the first aspect of the present invention.

The antibacterial fiber aggregate according to still another aspect of the present invention comprises a fiber aggregate impregnated with an antiseptic or bactericide of the first aspect.

The antibacterial fiber aggregate may be one whose fiber surface carries an antiseptic or bactericide according to the first aspect.

The method for eradicating a microorganism according to still another aspect of the present invention uses an amide group-containing guanidine derivative represented by the General Formula (I) or a salt thereof to eradicate a microorganism.

The method for inhibiting the proliferation of a microorganism according to still another aspect of the present invention uses an amide group-containing guanidine derivative represented by the General Formula (I) or a salt thereof.

Other objects and features of this invention will become apparent from the following description.

The bactericide or antiseptic according to the present invention contains as its active ingredient an amido group-containing guanidine derivative. A dermally applicable composition, washing composition or antibacterial fiber aggregate utilizes bactericides and/or antiseptic according to the invention. The present invention is detailed below in the order shown below.
[I] Amide group-containing guanidine derivative,
[II] Bactericide and antiseptic containing amide group-containing guanidine derivative,
[III] Dermally applicable composition,
[IV] Washing composition, and
[V] Antibacterial fiber aggregate.

### [I] Amide group-containing guanidine derivative:

An amide group-containing guanidine derivative employed in the invention is represented by the General Formula (I) shown below. 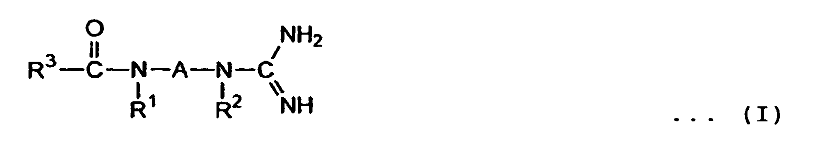 wherein each of R¹ and R² denotes a hydrogen atom, a straight or branched alkyl group or alkenyl group having 1 to 4 carbon atoms. R¹ and R² are the same or different. Most preferably, each of R¹ and R² is hydrogen atom.

In the General Formula (I), an acyl group (R³CO) may for example be a straight or branched, saturated or unsaturated acyl group having 12 to 14 carbon atoms. Typically, the acyl group (R³CO) may for example be an acyl group derived from lauric acid, or myristic acid.

In the General Formula (I), A is a branched or straight alkylene group or alkenylene group having 1 to 10, preferably 2 to 6 carbon atoms. Typically, A may for example be an ethylene group, propylene group, butylene group, pentylene group and hexylene group.

An amino group-containing guanidine derivative is incorporated usually as a salt, which is typically an inorganic acid salt such as a hydrochloride, sulfate, phosphate and the like, as well as an organic acid salt such as a glycolate, acetate, citrate, acidic amino acid salt, pyrrolidone carboxylate, fatty acid salt, acylamino acid salt and the like. A single inorganic or organic acid may be employed, or a mixture of two or more acids may also be employed.

An amino group-containing guanidine derivative or its salt employed in the invention can be obtained for example by a method disclosed in Japanese Patent Application Laid-Open 6-312972 in which a monoamide amine derived from a diamine is treated under reduced pressure while heating, bubbled with nitrogen while heating, or stored under a carbon dioxide-free atmosphere, guanidylated with a cyanamide, S-methylisothiourea and the like, and then made free from any contaminating impurities by means of crystallization and the like.

It is also possible to effect the production by reacting an amino group-containing guanidine derivative represented by General Formula (II) with a fatty acid halide or fatty acid ester. 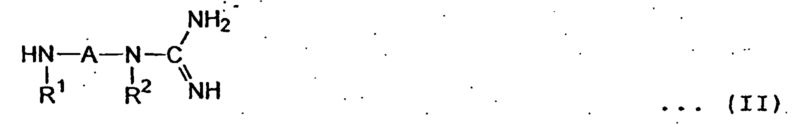 wherein each of R¹ and R² are same or different and each denotes a hydrogen atom, a straight or branched alkyl group or alkenyl group having 1 to 4 carbon atoms, and A denotes a straight or branched alkylene group or alkenylene group having 1 to 10 carbon atoms.

### [II] Bactericide and antiseptic containing amide group-containing guanidine derivative:

An amide group-containing guanidine derivative represented by the General Formula (I) or its salt has an excellent bactericidal effect and preserving effect against various microorganisms including E.coli, P.aeruginosa, S.aureus, S.mutans, P.acnes, C.albicans, A.niger, T.mentagrophytes, M.furfur, Methicillin-Resistant Staphylococcus aureus MRSA, C.cladosporioides and the like. The active ingredient content and dose of the amide group-containing guanidine derivative may vary widely depending on the dosage form, the types of microorganisms to be subjected and the time and duration of its occurrence and the like.

Usually, the concentration of an amide group-containing guanidine derivative represented by the General Formula (I) when used as a bactericide or antiseptic is 0.001 to 10 % by weight. However, in some certain cases, the concentration may exceed or may be below the range specified above. For example, a further lower concentration may be used when a synergistic effect can be obtained by means of a combination with other bactericides or antiseptics.

### [III] Dermally applicable composition:

A dermally applicable composition according to the invention contains an amide group-containing guanidine derivative represented by the General Formula (I) as a bactericide or antiseptic. An inventive dermally applicable composition may for example be a skin external formulation including a lotion, milky lotion, cream, makeup product (eye shadow, foundation, cream, lip color), fragrance, perfume, cologne, aromatic, deodorant, deodorizer, masking agent, antiperspirant, bath salt, insecticide, other skin care products, hair tonic, hair conditioner, hair make, hair cream, grease, hair restoration tonic, other hair care products, insect repelling spray, ointment and the like, to which an appropriate amount of an amide group-containing guanidine derivative is added to give a preserving ability or bactericidal activity, whereby increasing the commercial value.

In a dermally applicable composition of the invention, the amount of an amide group-containing guanidine derivative to be added is not particularly limited and may vary depending on the intended final product, but it is preferably 0.001 to 10 % by weight, particularly 0.01 to 5 % by weight. An amount less than 0.001 % by weight may allow the inventive effect to be exerted only insufficiently.

In addition to an amide group-containing guanidine derivative or its salt, various additives employed ordinarily in a dermally applicable composition may be contained as optional constituents in a dermally applicable composition of the invention as long as they do not affect the effect of the invention adversely. Examples are those listed in Standard of Cosmetic Ingredients, Standard of Cosmetic constituents by product types, Standard of Pharmaceuticals, Japanese Pharmacopoeia and Standards of Food Additives, such as surfactants, humectants, silicone compounds, polymers (polymeric compounds), alcohols, UV absorbers, dyes, pigments, vitamins, antioxidants, metal ion sequesters, anti-inflammatory agents, pH modifiers, pearly gloss imparting agents, nucleic acids, enzymes, natural extracts and the like. It is also possible to add other antiseptics and bactericides than amide group-containing guanidine derivatives represented by the General Formula (I).

The surfactants may for example be,

### 1. Anionic surfactants

Alkyl sulfates, alkyl phosphates, polyoxyethylene alkyl ether sulfates, polyoxyethylene alkylphenyl ether sulfates, polyoxyethylene alkyl carbonates, alkylphenylether sulfonates, salts of alkylsulfosuccinic acids and their derivatives, salts of N-acylsarcosine and their derivatives, N-acyl-N-methyl-β-alanine salts, polyoxyethylene palm oil fatty acid monoethanol amide sulfates, polyoxyethylene alkyl ether phosphates, higher fatty acid salts, N-acylglycine salts, N-acylalanine salts and the like,

### 2. Amphoteric surfactants

Carbobetaine-based amphoteric surfactants, sulfobetaine-based amphoteric surfactants, hydroxysulfobetaine-based amphoteric surfactants, amidosulfobetaine-based amphoteric surfactants, phosphobetaine-based amphoteric surfactants, imidazoline-based amphoteric surfactants, lecithin derivative and the like,

### 3. Nonionic surfactants

Propylene glycol fatty acid esters, glycerin fatty acid esters, polyoxyethylene glycerin fatty acid esters, polyglycerin fatty acid esters, sorbitan fatty acid esters, polyoxyethylene sorbitan fatty acid esters, polyoxyethylene sorbit fatty acid esters, polyoxyethylene alkylphenyl formaldehyde condensates, polyoxyethylene castor oil, polyoxyethylene hardened castor oil, polyoxyethylene sterol and its derivatives, polyethylene glycol fatty acid esters, polyoxyethylene alkyl ethers, polyoxyethylene polyoxypropylene alkyl ether, polyoxyethyene alkylphenyl ether, polyoxyethylene lanolin and its derivatives, polyoxyethylene beeswax derivatives, polyoxyethylene alkylamines, polyoxyethylene fatty acid amides, alkanol amides, sugar esters, polyoxyethylene hardened castor oil pyroglutamate esters, polyoxyethylene glyceryl pyroglutamate esters and the like,

### 4. Cationic surfactants

Quaternary ammonium salts, amide amines, N-acyl arginine ester salts, N-[3-acyloxy-2-hydroxypropyl]-L-arginine salt and the like.

A salt of an anionic surfactant may for example be sodium salts, magnesium salts, potassium salts, ammonium salts, diethanolamine salts, triethanolamine salts, arginine salts, lysine salts and the like, and these various surfactants may also be added.

The humectants may for example be,

### 1. Polyhydric alcohols

Glycerin, propylene glycol, 1,3-butylene glycol, isoprene glycol, polyethylene glycol, diglycerin, triglycerin, tetraglycerin, pentaglycerin, hexaglycerin, decaglycerin, sorbitol, ethylene glycol, erythritol and the like,

### 2. Natural moisturizing factors (NMFs)

Amino acids such as proline, glycine, alanine, serine, threonine, arginine, glutamic acid, aspartic acid, leucine, isoleucine and valine, pyrrolidone carboxylic acid or pyrrolidone carboxylate, lactic acid or lactates, urea, glucosamine, creatine, citrates and the like,

### 3. Saccharides, polysaccharides and polysaccharide derivatives

Sucrose, lactose, trehalose, natural oligosaccharides, hyaluronic acid and their sodium salts, chondroitin sulfate and the like,

### 4. Polyamino acids

Polyamino acids comprising polyglutamic acids and polyaspartic acids and their salts, as well as amino acid derivatives, soybean protein decomposition products, deoxyribonucleic acid, glycine betaine, chitin, chitosan, deacetylated chitin and the like.

The silicone compounds may for example be,

### 1. Ether-modified silicones

Methylpolysiloxanes, highly polymerized methylpolysiloxanes, polyoxyethylene-methylpolysiloxane copolymers, polyoxypropylene-methylpolysiloxane copolymers and poly(oxyethylene, oxypropylene)-methylpolysiloxane copolymers and the like,

### 2. Amino-modified silicones

Stearoxymethylpolysiloxane, stearoxytrimethylsilane, methyl hydrogen polysiloxane, octamethylpolysiloxane, decamethylpolysiloxane, cyclic silicones such as decamethylcyclopentasiloxane, octamethylcyclotetrasiloxane, tetrahydrotetramethylcyclotetrasiloxane, methylcyclopolysilonane and dodecamethylcyclohexasiloxane, methylphenylpolysiloxane, trimethylsiloxysilicic acid, aminoethylaminopropylsiloxane-dimethylsiloxane copolymers and the like,

### 3. Others

Silanol-modified polysiloxanes, alkoxy-modified polysiloxanes, fatty acid-modified polysiloxanes, fluorine-modified polysiloxanes, epoxy-modified polysiloxanes, alkoxy-modified polysiloxanes perfluoropolyethers, polyvinyl acetate dimethylpolysiloxane and the like.
In addition, a silicone emulsion obtained by emulsifying one or more of those listed above may also be employed.

The polymers (polymeric compounds) may for example be,

### 1. Vegetable polysaccharide polymers

Guar gum, locust bean gum, quince seed, carrageenin, galactan, gum arabic, tragacanth gum, pectin, mannan, starch, pullulan and the like,

### 2. Microorganism-derived polysaccharide polymers

Xanthane gum, dextran, succinoglucan, curdlan, hyaluroic acid and the like,

### 3. Animal proteins

Gelatin, casein, albumin, collagen and the like,

### 4. Cellulose derivatives

Methyl cellulose, ethyl cellulose, hydroxyethyl cellulose, hydroxypropyl cellulose, carboxymethyl cellulose and its salts, methylhydroxypropyl cellulose and the like,

### 5. Starch derivatives

Soluble starches, carboxyl starch, methyl starch and the like,

### 6. Alginic acid derivatives

Alginic acid propylene glycol.ester, alginates and the like,

### 7. Vinylic derivatives

Polyvinyl alcohol, polyvinyl butyral, vinyl acetate-vonyl pyrrolidone copolymer, vinyl acetate-crotonic acid copolymer, vinylmethyl ether-ethyl maleate copolymer, vinylmethyl ether-butyl maleate copolymer, crotonic acid-vinyl acetate- vinyl neodecanoate copolymer, methoxyethylene maleic anhydride copolymer, isobutylene-sodium maleate copolymer, N-methylpyrrolidone, vinylpyrrolidone-N,N-dimethylaminoethylmethacrylic acid copolymer diethylsulfate, vinylimidazolium methocloride-vinylpyrrolidone copolymer, polyvinylpyrrolidone, vinylpyrrolidone-styrene copolymer, vinylpyrrolidone-hexadecene copolymer, styrene-vinylpyrrolidone copolymer, eicosene-vinylpyrrolidone copolymer, carboxyvinylpolymer and the like,

### 8. (Meth)acrylic acid derivatives

Alkyl acrylate copolymer, polyacrylic acid and its salts (sodium, potassium, ammonium, triethanolamine, arginine, lysine and the like), alkyl polyacrylates, alkyl acrylate copolymers, acrylic acid amide- styrene copolymer, alkyl acrylate styrene copolymer, acrylic acid octyl amide-acrylic acid ester copolymer and its salts, acrylic acid octylamide-hydroxypropyl acrylate-butylaminoethyl methacrylate copolymer, acryl resin alkanolamines, hydroxyerhyl acrylate-methoxyethyl acrylate copolymer, acrylic acid alkyl ester-methacrylic acid alkyl ester-diacetone-acetone acrylamide-methacrylic acid copolymer, dimethyldiallylammonium chloride-acrylamide copolymer and the like, methacryloylethyldimethylbetaine -methacryloylethyltrimethylammonium chloride-methacrylic acid methoxypolyethylene glycol copolymer, methacryloylethyldimethylbetaine-methacryloylethyltrime thylammonium chloride-2-hydroxyethyl methacrylate copolymer,

### 9. Cationic celluloses

Chlorinated O-[2-hydroxy-3-(trimethylammonio)propyl]hydroxyethyl cellulose, chlorinated O-[2-hydroxy-3-(lauryldimethylammonio)propyl]hydroxyeth yl cellulose and the like,

### 10. Cationic guar gums

Chlorinated O-[2-hydroxy-3-(trimethylammonio)propyl]guar gum and the like,

### 11. Others

Carboxymethyl dextran and its salts, epoxy resin isostearic acid ester, polyamide epichlorohydrin resin, bisphenol epoxy resin fatty esters, polyethylene glycol epichlorohydrin palm oil alkylamine-dipropylene triamine condensate, perfluoropolyether and the like.

The alcohols may for example be higher alcohols such as cetyl alcohol, stearyl alcohol, behenyl alcohol, isostearyl alcohol, octyl dodecanol, oleyl alcohol, myristyl alcohol and the like, as well as lower alcohols such.as ethanol, isopropyl alcohol and the like.

The UV absorbers may for example be benzophenone derivatives, p-aminobenzoic acid derivatives, methoxycinnamic acid derivatives, salicylic acid derivatives, urocanic acid derivatives, 4-tert-butyl-4'-methoxydibenzoylmethane, 2-(2'-hydroxy-5'-methylphenyl)benzotriazole,methyl anthranilate and the like.

The dyes may for example be Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1 and the like.

The pigments may for example be natural pigments such as β-carotene, calcamine, cochineal and the like, inorganic pigments such as mica, talc, kaolin, calcium carbonate, magnesium carbonate, silicic anhydride, aluminium oxide, barium sulfate, colcothar, iron oxide yellow, iron oxide black, chromium oxide, ultramarine, prussian blue, carbon black, titanium oxide, zinc oxide, mica titanium, fish scale powder, bismuth oxychloride, boron nitride, photochromic pigments, synthetic fluorinated gold mica, iron-containing synthetic fluorinated gold mica, microparticulate composite powders and the like.

The vitamins may for example be those employed as nutrients such as vitamins A, B1, B2, B6 and their derivatives, those employed as whitening agents such as vitamin C and its derivatives, those employed as vasodilators such as vitamin E and its derivatives and the like.

The antioxidants may for example be tocopherols, dibutylhydroxytoluene (BHT), butylhydroxyanisol (BHA) and the like.

The metal ion sequesters may for example be ethylenediamine tetraacetic acid (EDTA) sodium salt, phosphoric acid, citric acid, succinic acid, gluconic acid, polyphosphoric acid, sodium methaphosphate and the like.

Examples of antiseptics and bactericides other than amide group-containing guanidine derivatives represented by the General Formula (I) may for example be benzoic acid, sodium benzoate, isopropylmethylphenol, zinc undecylenoate, undecylene monoethanol amide, zinc chloride, benzalkonium chloride, alkyltrimethylammonium chloride, cetylpyridinium chloride, benzalkonium chloride, alkyldiaminoethylglycine hydrochlorides, chlorhexidine hydrochloride, O-phenylphenol, photosensitive element No. 101, photosensitive element No. 201, chlorhexidine gluconate, cresol, chloramine T, chlorxylenol, chlorcresol, chlorphenesin, chlorobutanol, salicylic acid, sodium salicylate, alkylisoquinolinium bromide, domiphen bromide, sorbic acid and its salts, thymol, thiram, dehydroacetic acid and sodium, trichlorocarbanilide, p-oxybenzoates, chlorophenol, halocarban, phenol, hexachlorophene, resorcine, trichlosan, benzethonium chloride and the like.

The anti-inflammatory agents may for example be β-glycyrrhezic acid, glycyrrhizic acid derivatives, allantoin, azulene, ε-aminocaproic acid, hydrocortisone, hinokithiol and the like.

Otherwise, whitening agents such as albutin and kojic acid, trichogenic agents such as swertia japonica extract, cepharanthine, γ-orizanol, capsicum tincture, zingiberis rhizoma tincture and nicotinic acid benzyl ester, female hormones such as estradiol and ethynylestradiol, hair root activators such as panthotenic acid and its derivatives, placenta extract, allantoin, photosensitive element No. 301 may be exemplified.

Dry skin preventing agents such as zinc oxide, zinc sulfate, allantoin hydroxyaluminium, aluminium chloride, aluminium sulfate, zinc sulfocalbolate and tannic acid and refreshing agents such as menthol and camphor may also be exemplified.

In addition, antihistaminic agents such as diphenhydramine hydrochloride and chlorphenylamine maleate, and keratin peeling - keratolytic agents such as sulfur, salicylic acid and resorcine may also be exemplified.

Those which can also be exemplified are naturally occurring drugs such as essential oils of hamamelis, sage, white birch, Rhei Rhizoma, glycyrrhizal radix, copridis rhizoma, chicory, achillea millefolium, aloe, chamomilla and eucalyptus, as well as carrot extract, aloe, chicory, lily, loofah, horse chestnut, phellodendri Cortex, Safflower and the like.

Those which can further be exemplified are hydrolyzed proteins such as hydrolyzed protein collagen and hydrolyzed silk, repellents against hematophagous insects (mosquito, louse, flea, mite and the like) such as dimethyl phthalate, 2-ethyl-1,3-hexanediol, bisbutylene tetrahydrofurfural, N,N-diethyl-m-toluamide and the like.

The dosage form of a dermally applicable composition is not limited particularly, and may be any form such as an emulsion, solution, solubilized system, powder dispersion, water-oil biphasic system, water-oil-powder triphasic system, and the like.

### [IV] Washing composition:

A washing composition according to the invention is detailed below. An inventive washing composition contains as a bactericide or antiseptic an amide group-containing guanidine derivative represented by the General Formula (I) . The inventive washing composition may for example be a powder dentifrice, paste dentifrice, mouth washer (liquid dentifrice, mouth washer, rinse), other oral cleansing agents, shampoo, rinse, other hair cleansing agents, body soap, face washer, make up cleanser, soap, other skin cleansing agents, dish washing agent, laundry cleanser, softener, disinfecting cleanser, deodorizing cleanser, furniture care products, bleaching agent and the like, to each of which an amide group-containing guanidine derivative in an appropriate amount is added whereby imparting the product with the preserving ability and the bactericidal activity, whereby increasing the commercial value. A product especially in the form of an oral composition is imparted frequently with a certain function or efficacy on the oral cavity such as a bactericidal effect, and an amide group-containing guanidine derivative represented by the General Formula (I) can be employed as an antiseptic or bactericide against oral microorganisms.

While the amount of an amide group-containing guanidine derivative represented by the General Formula (I) in a washing composition according to the invention is not limited particularly and may vary depending on the intended final product, it is preferably 0.001 to 10 % by weight, particularly 0.01 to 5 % by weight. An amount less than 0.001 % by weight may allow the effect of the invention to be exerted only insufficiently.

In addition to an amide group-containing guanidine derivative represented by the General Formula (I) or its salt, various additives employed ordinarily in a washing composition may be incorporated as optional components as long as the effect of the invention is not affected adversely. Examples of such additives are a bactericide or antiseptic other than an amide group-containing guanidine derivatives represented by the General Formula (I), chelating agents, surfactants, fragrances, sweeteners, bonds, humectants, abrasives, pH modifiers, other pharmacologically effective substances and the like.

The bactericide or antiseptic other than an amide group-containing guanidine derivatives represented by the General Formula (I) may for example be p-oxybenzoic acid and p-oxybenzoates (e.g., methyl p-oxybenzoate, ethyl p-oxybenzoate), cetylpyridinium chloride, benzalkonium chloride, sodium methyl p-oxybenzoate, benzyl o-oxybenzoate, p-phenolsulfonic acid and its salts (e.g., sodium p-phenolsulfonate), phenol, p-chlorophenol, p-chlorometacresol, p-chlorometaxylenol, dicloroxylenol, isopropylmethylphenol, resorcine, resorcine monoacetate, o-phenylphenol, thiobischlorophenol, sodium o-phenol, sodium phenoxide, chlorophenesin, phenoxyethanol, thymol, chlorothymol, phenols such as pyrogallol, cresol, hinokithiol and hydroxybenzothiol, benzoic acid and its salts, salicylic acid and its salts, acids such as dehydroacetic acid and its salts, sorbic acid and its salts, boric acid and the like, halogenated bisphenols such as hexachlorophenone and 2,4,4'-trichloro-2'-hydroxydiphenyl ether, amides such as 3,4,4'-trichlorocarbanilide, 3-trifluoromethyl-4,4'-dichlorocarbanilide, undecylenic acid monoethanolamide, chloroacetamide and the like, domiphen bromide, alkylisoquinolium bromides, alkyltrimethylammonium bromides, cetyltrimethylammonium saccharin, methylbenzethonium chloride, laurylpyridinium chloride, laurylcholaminoformylmethylpyridinium, decanium chloride, stearyldimethylbenzylammonium chloride, quaternary ammonium compounds such as benzethonium chloride, alkyltrimethylammonium chlorides and the like, amphoteric compounds such as lauryldi(aminoethyl)glycine, laurylaminoethylglycine, alkyldiaminoethylglycine hydrochloride and the like, chlorhexidine, diisethionic acid dibromopropamidine, chlorhexidine gluconate, phenylethyl alcohol, benzyl alcohol, dochlorobenzyl alcohol, glutardialdehyde, chloramine T, zinc pyrithione, sodium pyrithione, furfural, PLATONIN, pionin, LUMINEX, p-dimethylaminostyrylheptylmethylthiazonium iodide, 5-bromo-5-nitro-1,3-dioxane, tetramethylthiuram disulfide, 1-hydroxypyridine-2-thione, imidazoylurea compounds, N-trichloromethyl mercapto-4-cyclohexene-1,2-dicarboxyimide, lyzozyme chloride, chlorobutanol, 2-bromo-2-nitro-1,3-propanediol, 6-acetoxy-2,4-dimethyl-m-dioxane, diethyl pyrocarbonate, ethylene oxide, β-propionelactone, any of which may be employed alone or in combination with each other.

The chelating agents may for example be hydroxyethylethylene diamine triacetate, disodiumedetate, gluconodeltalacton, gluconic acid, sodium polyphosphate, alanine, sodium citrate and the like.

The surfactants may for example be the surfactants exemplified above in the section of [III] Dermally applicable composition.

The fragrances may for example be menthol, carbon, anethol, eugenol, methyl salicylate, limonene, ocimene, n-decyl alcohol, citronel, α-terpineol, methyl acetate, citronenyl acetate, methyl eugenol, cinneol, linalol, ethyl linalol, vaniline, thymol, speamint oil, peppermint oil, lemon oil, orange oil, sage oil, rosemary oil, cinnamon oil, beefsteak plant leaf oil, wintergreen oil, syzygium aromaticum oil, eucalyptus oil and the like, any of which may be employed alone or in combination with each other.

The sweeteners may for example be saccharin sodium, acesulfame potassium, stevioside, neohesperidyl dihydrocarcone, glycyrrhzin, perillartine, thaumatine, aspartylphenylalanylmethylester, p-methoxycinnamic aldehyde and the like, any of which may be employed alone or in combination.

The bonds may for example be carrageenan, cellulose derivatives such as carboxymethyl cellulose, alkali metal alginates such as sodium alginate, gums such as xanthane gum, tragacanth gum and gum arabic, synthetic bonds such as polyvinyl alcohol and sodium polyacrylate, inorganic bonds such as silica gel, aluminium silica gel, BEGUM (aluminium/magnesium silicate) and the like, any of which may be employed alone or in combination.

The humectants may for example be sorbitol, glycerin, ethylene glycol, propylene glycol, 1,3-butylene glycol, polyethylene glycol, polypropylene glycol, xylitol, maltitol, lactitol and the like, any of which may be employed alone or in combination.

The abrasives may for example be dicalcium hydrogen phosphate dihydrate and anhydride, calcium dihydrogen phosphate, tricalcium phosphate, calcium carbonate, calcium pyrophosphate, aluminium hydroxide, alumina, insoluble sodium metaphosphate, trimagnesium phosphate, magnesium carbonate, calcium sulfate, methyl polymethacrylate and synthetic resins and the like, any of which may be employed alone or in combination.

When an oral composition is intended, it may contain as an active ingredient a vitamine E such as dl-α-tocopherol acetate, tocopherol succinate, tocopherol nicotinate and the like, a nonionic bactericide such as trichlosan, an amphoteric bactericide such as dodecyldiaminoethylglycine, an enzyme such as dextranase, amylase, protease, mutanase, lyzozyme and lytic enzymes, an alkali metal monofluorophosphate such as sodium monofluorophosphate and potassium monofluorophosphate, a fluoride such as sodium fluoride and stannous fluoride, as well as tranexamic acid or ε-aminocaproic acid, aluminium chlorhydroxylallantoin, dihydrocholesterol, glycyrrhizic acid salts, glycyrrhezic acid, glycerophosphate, chlorophyll, sodium chloride, callopeptide, water-soluble inorganic phosphoric acid compounds, any of which may be employed alone or in combination.

The dosage form of a washing composition is not limited particularly, and may be any form such as an emulsion, solution, solubilized system, powder dispersion, water-oil biphasic system, water-oil-powder triphasic system, and the like.

### [V] Antibacterial fiber aggregate:

An antibacterial fiber aggregate of the invention is described below. The antibacterial fiber aggregate of the invention can broadly be classified into a wet-type obtained by impregnating a fiber aggregate with a drug solution of an amide group-containing guanidine derivative represented by the General Formula (I) or its salt and a dry-type having the amide group-containing guanidine derivative represented by the General Formula (I) or its salt as being supported on the surface of the fiber. The following fibers aggregate will be explained.
(i) wet-type antibaceterial fiber aggregate, and
(ii) dry-type antibaceterial fiber aggregate.

### (i) Wet-type antibaceterial fiber aggregate

A wet-type antibacterial fiber aggregate may for example be in the form of a product obtained by impregnation with a drug solution containing an amide group-containing guanidine derivative represented by the General Formula (I) or its salt followed by air-tight packaging with a plastic film, or followed by storage in a container from which the content is subsequently pulled out for use, as with a wet tissue product. Since such a wet tissue product is susceptible to a bacterial growth due to the storage in a wet condition, it is generally supplemented with an antiseptic or bactericide whereby preventing any bacteria growth.

A fiber aggregate used in a wet tissue product is sin a certain morphology as a result of the aggregation of the fibers, as is in a woven or non-woven fabric. Typically, a paper which is a non-woven fabric made from rayon or cellulose fibers, a cotton which is an aggregate of cotton fibers, a paper made from a pulp, a silk aggregate made from silk yarns, may be exemplified.

While the amount of an amide group-containing guanidine derivative in a drug solution employed in an inventive wet-type antibacterial fiber aggregate is not limited particularly, it is preferably 0.001 to 10 % by weight, particularly 0.01 to 5 % by weight. An amount less than 0.001 % by weight may allow the effect of the invention to be exerted only insufficiently.

In addition to an amide group-containing guanidine derivative or its salt, various additives employed ordinarily in a wet tissue product may be added as optional components to a drug solution employed in an inventive wet-type antibacterial fiber aggregate, as long as the effect of the invention is not affected adversely. Examples of the additives are surfactants, humectants, alcohols and the like, as well as antiseptics or bactericides other than the amide group-containing guanidine derivatives represented by the General Formula (I). Those exemplified typically are those exemplified in the section of [III] Dermally applicable composition. Those which may also be added are anti-inflammatory agents, fragrances, deodorizers and the like.

### (ii) Dry-type antibacterial fiber aggregate

A dry-type antibacterial fiber aggregate is a fiber aggregate whose fiber surface carries an amide group-containing guanidine derivative represented by the General Formula (I) or its salt, and one which may be exemplified is obtained by first impregnating the entire fiber aggregate with a drug solution containing the amide group-containing guanidine derivative represented by the General Formula (I) or its salt followed by drying, or by first spraying an amide group-containing guanidine derivative in a powder form onto the surface of a fiber made from a thermoplastic resin followed by heating and pressurizing whereby depositing the powder of the amide group-containing guanidine derivative on the surface of the fiber. Such a dry-type antibacterial fiber aggregate can be employed for example in an air filter of an air conditioner, for the purpose of inhibiting or preventing any bacterial growth on the air filter.

While the amount of an amide group-containing guanidine derivative to be carried on an inventive dry-type antibacterial fiber is not limited particularly, it is 0.1 to 100 g/l m².

A fiber aggregate employed in a dry-type antibacterial fiber aggregate may be same to those described in relation to a wet-type antibacterial fiber aggregate.

### Example 1

Embodiments of the present invention are further detailed, by way of illustration only in the following Examples, which are contrasted with Comparative Examples.

### (1) Synthesis of amide group-containing guanidine derivative

180g of water and 110g of 2-propanol were added to 52.7g (0.23mol) of 4-aminobutylguanidine sulfate. pH of the system was adjusted to be 11.0 by 27% sodium hydroxide aqueous solution and the temperature was adjusted to be 10 °C. In the system, 50.3g (0.23mol) of lauroyl chloride was added dropwise over 35minutes. At this time, the temperature of the system was kept at 8 to 12 °C, and pH was kept at 10.9 to 11.0 by the 27% sodium hydroxide aqueous solution. After completion of the dropwise addition, the reaction was further ripened over 30minutes.

After completion of reaction, the temperature of the system was adjusted to be 50 °C. The resulting oily phase was separated, adjusted to be pH 14 by 27% sodium hydroxide aqueous solution and cooled to room temperature. After cooling, the precipitated solid was separated by filtration and after it was further washed with water, it was dried.
Light yellow solid 65.8g (yield 92%)
¹H-NMR(CD3OD) : σ = 0.90(3H, t), 1.28-1.38(18H, m), 1.57(6H, m), 2.17(2H, t), 3.19(4H, m)
ESI-Mass: 313(MH+)

A peak was confirmed by High Performance Liquid Chromatography (column: YMC-Pack ODS-AMAM-312, S-5um, 120A, 150*6.0mm, column temperature: 40 °C, eluent: 30mM Phosphate buffer (pH3.0) / methanol = 25/75, flow rate:1.0ml/min, detection :UV210nm) .

0.1% of sulfate ion in lauroylamidobutylguanidine was confirmed by Ion Chromatography (column: Ion pac AS-11 HC 2mm (Dionex), guard column: Ion pac AG-11 HC 2mm (Dionex), column temperature: room temperature, eluent: NaOH(aq) 1.5mM (0min) → 30mM(20min), flow rate:0.38ml/min, recycle solution: 5mM H₂SO₄ about 3mL/min, detection: conductivity).

Amide group-containing guanidine derivatives having different acyl chain lengths employed in Examples were synthesized in the similar manner.

### (2) Antibacterial activity evaluation

4-Guanidinobutyldodecanamide hydrochloride (LAG-HCl) which is a salt of an amide group-containing guanidine derivative and, for comparison, N-cocoyl-L-arginine ethyl ester-DL-pyrrolidone carboxylate which is an N-long chain acyl basic amino acid derivative were examined for their concentrations at which the growth of each test strain was inhibited completely (MIC).

The test strains employed were Escherichia coli (ATCC8739), Pseudomonas aeruginosa (ATCC9027), Staphylococcus aureus (ATCC6538), Streptococcus mutans (ATCC35275), Propionibacterium acnes (ATCC6919), Candida albicans (ATCC10231), Aspergillus niger (ATCC16404), Trichophyton mentagrophytes (ATCC11480) and Malassezia furfur (ATCC14521).

A bacterial cell suspension of E.coli, P.aeruginosa, S.aureus and S.mutans was obtained by incubation in a sensitive broth medium at 35 °C for 20 hours . The bacterial cell suspension of P.acnes was obtained by anaerobic incubation in a GAM agar medium at 35 °C for 20 hours followed by collecting cells with a platinum loop spatula followed by suspending the cells in a sterilized physiological saline at a cell density at about 10⁸ cells/ml. The bacterial cell suspension of C.albicans was obtained by incubation in a potato dextrose agar medium at 25 °C for 48 hours followed by collecting cells with a platinum loop spatula followed by suspending the cells in a sterilized physiological saline at a cell density at about 10⁶ cells/ml. The bacterial cell suspension of A.niger was obtained by incubation in a potato dextrose agar medium at 25 °C for 7 days followed by collecting cells with a platinum loop spatula followed by suspending the cells in a sterilized physiological saline followed by filtration through a sterilized gauze to adjust at a cell density at about 10⁶ cells/ml. The bacterial cell suspension of T.mentagrophytes was obtained by incubation in a Sabouraud's agar medium at 25 °C for 7 days followed by collecting spores and hyphae with a platinum loop spatula followed by suspending the cells in a sterilized physiological saline followed by pelletizing using a homogenizer to adjust at a cell density at about 10⁶ cells/ml. The bacterial cell suspension of M.furfur was obtained by incubation in an YMO agar medium at 25 °C for 7 days followed by suspending the test strain in a sterilized phosphate-buffered physiological saline at a cell density at about 10⁶ cells/ml.

To obtain an antibacterial activity assay sample, 4-guanidinobutyldodecanamide hydrochloride was dissolved in ethylene glycol at 2 % which was used as a mother liquor which was diluted with water subsequently. N-cocoyl-L-arginine ethyl ester-DL-pyrrolidone carboxylate was dissolved in water to prepare a test solution. The respective minimum growth inhibition concentration at which no microbial growth was observed was determined (µg/ml).

For each test strain, the respective agar medium indicated in the table shown below was shown. The agar medium (19 ml) was dissolved once and then kept at 50 °C, and to this solution 1 ml of each diluted cell suspension was added until reaching the intended concentration and mixed thoroughly and then solidified.

Subsequently, the cell suspension of each test strain was sampled using a platinum loop spatula, with which an about 1 cm line was drawn on the antibacterial agent-supplemented agar plate. Each plate was incubated at the respective temperature for the respective time period shown below.

**Table 1:**

| Test strain | Culture medium | Incubation condition |
|---|---|---|
| E.coli | Muller-Hinton agar medium | 35 °C for 48 hours |
| P.aeruginosa | Muller-Hinton agar medium | 35 °C for 48 hours |
| S.aureus | Muller-Hinton agar medium | 35 °C for 48 hours |
| S.mutans | Muller-Hinton agar medium | 35 °C for 48 hours |
| P.acnes | GAM agar medium | 35 °C for 48 hours (anaerobic incubation) |
| C.albicans | Potato dextrose agar medium | 25 °C for 48 hours |
| A.niger | Potato dextrose agar medium | 25 °C for 5 days |
| T.mentagrophytes | Sabouraud's agar medium | 25 °C for 7 days |
| M.furfur | YMO agar medium | 25 °C for 7 days |

The following are the minimum growth inhibition concentration (MIC) (µg/ml) of 4-guanidinobutyldodecanamide hydrochloride (LAG-HCl) and N-cocoyl-L-arginine ethyl ester-DL-pyrrolidone carboxylate (CAE).

**Table 2:**

| Test strain | MIC(ug/ml) | |
|---|---|---|
| | Example 1 | Comparative 1 |
| | LAG-HCl | CAE |
| E.coli | 75-100 | 75-100 |
| P.aeruginosa | 75-100 | 200-300 |
| S.aureus | 7.5-10 | 5-7.5 |
| S.mutans | 5-7.5 | 5-7.5 |
| P.acnes | 7.5-10 | 20-30 |
| C.albicans | 500-750 | 300-500 |
| A.niger | 200-300 | 300-500 |
| T.mentagrophytes | 75-100 | 100-150 |
| M.furfur | 100-150 | 75-100 |

As shown in Table 2, a satisfactory antibacterial activity against any of the test strains was observed with both of 4-guanidinobutyldodecanamide hydrochloride (LAG-HCl) and N-cocoyl-L-arginine ethyl ester-DL-pyrrolidone carboxylate (CAE).

As described above, the amide group-containing guanidine derivative exhibited an excellent antibacterial activity equivalent to that of N-cocoyl-L-arginine ethyl ester-DL-pyrrolidone carboxylate (CAE).

### (3) Chemical stability evaluation

In order to evaluate the chemical stability of 4-guanidinobutyldodecanamide hydrochloride (LAG-HCl), 0.0497 g of 4-guanidinobutyldodecanamide hydrochloride (LAG-HCl) was dissolved in 50 ml of water and stored at 50 °C for 2 weeks. After the 2-week storage period, the sample was analyzed by HPLC, the data of which was compared with the HPLC data before storage, and the results indicated that there was no new peaks or no change in the peak areas after the storage, revealing that 4-guanidinobutyldodecanamide hydrochloride (LAG-HCl) is highly stable in an aqueous solution.

These findings may be due to the fact that, unlike N-long chain acyl basic amino acid derivatives such as CAE, 4-guanidinobutyldodecanamide hydrochloride (LAG-HCl) has no ester group in its molecule which is susceptible to hydrolysis.

### Examples 2 to 5

In order to evaluate the contribution of acyl moiety to the antibacterial activity in amido group-containing guanidine derivatives, 4-guanidinobutyl decanamide acetate (DAG-AcOH), 4-guanidinobutyldodecanamide acetate (LAG-AcOH), 4-guanidinobutymyristilamide acetate (MAG-AcOH), 4-guanidinobutylpalmitylamide acetate (PAG-AcOH) were examined for their concentrations at which the growth of each test strain was inhibited completely (MIC).

The test strains employed were Escherichia coli (ATCC8739) , Pseudomonas aeruglnosa (ATCC9027), Staphylococcus aureus (ATCC6538), Streptococcus mutans (ATCC35275) , Propionibacterium acnes (ATCC6919), Candida albicans (ATCC10231), Aspergillus niger (ATCC16404), Trichophyton mentagrophytes (ATCC11480), Malassezia furfur (ATCC14521), MRSA and Cladosporium cladosporioides (IF06348).

A bacterial cell suspension of E.coli, P.aeruginosa, S.aureus, S.mutans, P.acnes, C.albicans, A.niger, T.mentagrophytes, M. furfur was obtained as described above.

The bacterial cell suspension of MRSA was obtained by incubation in a sensitive broth medium at 35 °C for 20 hours.

The bacterial cell suspension of Cladosporium cladosporioides was obtained by incubation in a potato dextrose agar medium at 25 °C for 7 days followed by collecting cells with a platinum loop spatula followed by suspending the cells in a sterilized physiological saline followed by filtration through a sterilized gauze to adjust at a cell density at about 10⁶ cells /ml.

To obtain an antibacterial activity assay sample, each compound was dissolved in water to prepare a test solution, which was used as a mother liquor which was diluted with water subsequently.

The respective minimum growth inhibition concentration at which no microbial growth was observed was determined (Table 4).

For E.coli, P.aeruginosa, S.aureus, S.mutans, P.acnes, C.albicans, A.niger, T.mentagrophytes, M.furfur, the respective agar medium indicated in the Table 1 shown above was shown.

For MRSA and Cladosporium cladosporioides, the respective agar medium indicated in the Table 3 shown below was shown.

The agar medium (19ml) was dissolved once and then kept at 50°C, and to this solution 1ml of each diluted cell suspension was added until reaching the intended concentration and mixed thoroughly and solidified.

Subsequently, the cell suspension of each test strain was sampled using a platinum loop spatula, with which an about 1 cm line was drawn on the antibacterial agent-supplemented agar plate. Each plate was incubated at the respective temperature for the respective time period shown below.

**Table 3:**

| Test strain | Culture medium | Incubation condition |
|---|---|---|
| MRSA | Muller - Hinton agar medium | 35°C for 48 hours |
| C.cladospor ioides | Potato dextrose agar medium | 25°C for 5 days |

The followings are the minimum growth inhibition concentration (MIC) (µg /ml) of 4-guanidinobutyl decanamide acetate (DAG-AcOH) (comparative example), 4-guanidinobutyldodecanamide acetate (LAG-AcOH), 4-guanidinobutymyristilamide acetate (MAG-AcOH), 4-guanidinobutylpalmitylamide acetate (PAG-AcOH) (comparative example).

**Table 4:**

| test strain | MIC(ug/ml) | | | |
|---|---|---|---|---|
| | Example2 | Example3 | Example4 | Example5 |
| | for comparison DAG-AcOH | LAG-AcOH | MAG-AcOH | for comparison PAG-AcOH |
| E.coli | 150-200 | 30-50 | 50-75 | >10000 |
| P.aeruginosa | 200-300 | 75-100 | 300-500 | >10000 |
| S.aureus | 30-50 | 2-3 | 3-5 | 1500-2000 |
| MRSA | 50-75 | 10-15 | 10-15 | 1000-1500 |
| S.mutans | 20-30 | 5-7.5 | 3-5 | 30-50 |
| P.acnes | 30-50 | 10-15 | 10-15 | 500-750 |
| C.albicans | 300-500 | 300-500 | 500-750 | >10000 |
| A.niger | 300-500 | 200-300 | 300-500 | >10000 |
| C.cladosporioides | 200-300 | 150-200 | 200-300 | 7500-10000 |
| T.mentagrophytes | 75-100 | 30-50 | 75-100 | 500-750 |
| M.furfur | 50-75 | 50-75 | 100-150 | >10000 |

As shown in Table 4, a satisfactory antibacterial activity against any of the test strains was observed with 4-guanidinobutyldecanamide acetate (DAG-AcOH), 4-guanidinobutyldodecanamide acetate (LAG-AcOH), 4-guanidinobutymyristilamide acetate (MAG-AcOH), 4-guanidinobutylpalmitylamide acetate (PAG-AcOH).

As described above, the amide group-containing guanidine derivatives examined exhibited an excellent antibacterial activity. Among them, LAG-AcOH had the best antibacterial activity.

### Examples 6 and 7

The antibacterial lotions having the formulations (% by weight based on net composition, 100 % by weight in total) indicated in Table 5 shown below were prepared. When each antibacterial lotion thus obtained was used, a satisfactory performance was obtained.

**Table 5:**

| Antibacterial lotions | | |
|---|---|---|
| | Example 6 | Example 7 |
| 3-Guanidinopropylmyristylamide acetate | 0.10 | |
| 4-Guanidinobutylmyristylamide phosphate | | 0.10 |
| 1,3-Butylene glycol | 6.00 | 6.00 |
| Glycerin | 4.00 | 4.00 |
| Oleyl alcohol | 0.10 | 0.10 |
| POE(20) sorbitan monolauric acid ester | 0.50 | 0.50 |
| POE(15) lauryl alcohol ether | 0.50 | 0.50 |
| Ethanol | 10.00 | 10.00 |
| dl-Tocophenol | 0.20 | 0.20 |
| Sorbic acid | 0.20 | 0.20 |
| Kathon CG | 0.01 | 0.01 |
| Benzalkonium chloride | 0.02 | |
| Benzethonium chloride | | 0.02 |
| Methyl p-oxybenzoate | 0.10 | 0.10 |
| Purified water | Remainder | Remainder |

### Examples 8 to 10

The antibacterial milky lotions having the formulations (% by weight based on net composition, 100 % by weight in total) indicated in Table 6 shown below were prepared. When each antibacterial milky lotion thus obtained was used, a satisfactory performance was obtained.

**Table 6:**

| Antibacterial milky lotions | |
|---|---|
| | Example 8 |
| 4-Guanidinobutyldodecanamide acetate | 0.20 |
| Cetyl alcohol | 1.00 |
| Beeswax | 0.50 |
| Petrolatum | 2.00 |
| Squalane | 6.00 |
| Dimethylpolysiloxane | 2.00 |
| Ethanol | 5.00 |
| Glycerin | 4.00 |
| 1,3-Butylene glycol | 4.00 |
| POE(10) Monooleic acid ester | 1.00 |
| Glycerol monooleic acid ester | 1.00 |
| Quince seed extract (5 %) | 20.0 |
| Trichlosan | 0.1 |
| Phenoxyethanol | |
| Hinokithiol | |
| Trichlorocarbanilide | 0.1 |
| Methyl p-oxybenzoate | 0.30 |
| Butyl p-oxybenzoate | 0.05 |
| Purified water | Remainder |

### Examples 9 and 10

The antibacterial creams having the formulations (% by weight based on net composition, 100 % by weight in total) indicated in Table 7 shown below were prepared. When each antibacterial cream thus obtained was used, a satisfactory performance was obtained.

**Table 7:**

| Antibacterial creams | | |
|---|---|---|
| | Example 9 | Example 10 |
| 2-Guanidinoethylmyristylamide gluconate | 0.20 | |
| 4-Guanidinobutyldodecanamide | | 0.20 |
| Stearyl alcohol | 6.00 | 6.00 |
| Stearic acid | 2.00 | 2.00 |
| Hydrogenated lanolin | 4.00 | 4.00 |
| Squalane | 9.00 | 9.00 |
| Octyl dodecanol | 10.00 | 10.00 |
| 1,3-Butylene glycol | 6.00 | 6.00 |
| PEG1500 | 4.00 | 4.00 |
| POE(25) Cetyl alcohol ether | 3.00 | 3.00 |
| Glyceryl monostearate | 2.00 | 2.00 |
| Isopropylmethylphenol | 0.20 | 0.20 |
| Germal II | 0.20 | 0.20 |
| Paraben | 0.05 | 0.05 |
| Fragrance | Appropriate quantity | Appropriate quantity |
| Purified water | Remainder | Remainder |

### Examples 11 to 13

The antibacterial rinses having the formulations (% by weight based on net composition, 100 % by weight in total) indicated in Table 8 shown below were prepared. When each antibacterial rinse thus obtained was used, a satisfactory performance was obtained.

**Table 8:**

| Antibacterial rinses | | | |
|---|---|---|---|
| | Example 11 | Example 12 | Example 13 |
| 6-Guanidinohexyldodecanamide | 0.10 | | |
| 6-Guanidinohexylmyristylamide hydrochloride | | 0.10 | |
| 4-Guanidinobutylmyristylamide acetate | | | 0.10 |
| Silicone oil | 3.00 | 3.00 | 3.00 |
| Liquid paraffin | 1.00 | 1.00 | 1.00 |
| Cetyl alcohol | 1.50 | 1.50 | 1.50 |
| Stearyl alcohol | 1.00 | 1.00 | 1.00 |
| Stearyltrimethylammonium Chloride | 0.70 | 0.70 | 0.70 |
| Glycerin | 3.00 | 3.00 | 3.00 |
| Preservative | Appropriate quantity | Appropriate quantity | Appropriate quantity |
| Purified water | Remainder | Remainder | Remainder |

### Examples 14 to 17

The drug solutions having the formulations (% by weight based on net composition, 100 % by weight in total) indicated in Table 9 shown below were used to impregnate cellulose fiber non-woven fabrics to produce wet tissue products. When each wet tissue product thus obtained was used, a satisfactory performance was obtained.

**Table 9:**

| Wet tissue products | | | | |
|---|---|---|---|---|
| | Example 14 | Example 15 | Example 16 | Example 17 |
| (Drug solution) | | | | |
| 4-Guanidinobutyldodecanamide acetate | 0.2 | | 0.1 | 0.05 |
| 6-Guanidinohexyldodecanamide aspartate | | 0.05 | | |
| Lauryldimethylbenzylammonium Chloride | 0.1 | 0.1 | 0.1 | 0.1 |
| Paraben | 0,05 | 0.05 | 0.05 | 0.05 |
| Sodium salicylate | 0.1 | | | |
| Potassium sorbate | | | | |
| Sodium benzoate | | 0.1 | | |
| Sodium pyrrolidone carbonate | 0.2 | 0.2 | 0.2 | 0.2 |
| Propylene glycol | 3.00 | 3.00 | 3.00 | 3.00 |
| Purified water | Remainder | Remainder | Remainder | Remainder |
| Support | Cellulose fiber non-woven fabric | | | |

### Examples 18 and 19.

The drug solutions having the formulations (% by weight based on net composition, 100 % by weight in total) indicated in Table 10 shown below were used to impregnate rayon non-woven fabrics to produce wet sheet products. When each wet sheet product thus obtained was used, a satisfactory performance was obtained.

**Table 10:**

| Wet sheet products | | |
|---|---|---|
| | Example 18 | Example 19 |
| (Drug solution) | | |
| 4-Guanidinobutyldodecanamide acetate | 0.2 | |
| 2-Guanidinoethyldodecanamide | | 0.2 |
| Isobutylmethylphenol | 0.10 | 0.10 |
| Benzalkonium'chloride | 0.10 | 0.10 |
| Paraben | 0.10 | 0.10 |
| Glycerin | 2.00 | 2.00 |
| Aloe extract | 4.00 | 4.00 |
| Purified water | Remainder | Remainder |
| Support | Rayon non-woven fabric | |

### Examples 20 and 21

The drug solutions having the formulations (% by weight based on net composition, 100 % by weight in total) indicated in Table 11 shown below were used to impregnate cellulose fiber non-woven fabrics to produce wet tissue products. When each wet tissue product thus obtained was used, a satisfactory performance was obtained.

**Table 11:**

| Wet tissue products | | |
|---|---|---|
| | Example 20 | Example 21 |
| (Drug solution) | | |
| 4-Guanidinobutyldodecanamide hydrochloride | 0.10 | |
| 2-Guanidinoethyldodecanamide citrate | | 0.10 |
| Propylene glycol | 2.00 | 2.00 |
| Chitosan | 1.00 | 1.00 |
| Maltitol | 2.00 | 2.00 |
| Alkyldiaminoethylglycine hydrochloride | 0.10 | 0.10 |
| Dipotassium glycyrrhizinate | 0.20 | 0.20 |
| Laurylbenzyldimethylammonium chloride | 0.01 | 0.01 |
| Purified lecithin | 0.20 | 0.20 |
| Fragrance | Appropriate quantity | Appropriate Quantity |
| Purified water | Remainder | Remainder |
| Support | Cellulose fiber non-woven fabric | |

### Examples 22 to 23

The tooth pastes having the formulations (% by weight based on net composition, 100 % by weight in total) indicated in Table 12 shown below were prepared. When each tooth paste thus obtained was used, a satisfactory performance was obtained.

**Table 12.**

| Tooth pastes | | |
|---|---|---|
| | Example 22 | Example 23 |
| 4-Guanidinobutyldodecanamide acetate | 0.50 | |
| 4-Guanidinobutyldodecanamide gluconate | | 0.50 |
| Dicalcium phosphate dihydrate | 45.00 | 45.00 |
| Silicic anhydride | 2.00 | 2.00 |
| Glycerin | 15.00 | 15.00 |
| Sodium carboxymethyl cellulose | 1.00 | 1.00 |
| Carrageenin | 0.30 | 0.30 |
| Sodium lauryl sulfate | 1.50 | 1.50 |
| Sodium cocoyl glutamate | 0.50 | 0.50 |
| Sodium saccharate | 0.10 | 0.10 |
| Cetylpyridinium chloride | 0.01 | 0.01 |
| Laurylpyridinium chloride | 0.01 | 0.01 |
| Chlorhexidine hydrochloride | 0.01 | 0.01 |
| Stearyldimethylbenzylammonium Chloride | 0.01 | 0.01 |
| Fragrance | Appropriate quantity | Appropriate quantity |
| Ethyl p-oxybenzoate | 0.01 | 0.01 |
| Purified water | Remainder | Remainder |

### Examples 24 to 25.

The clear tooth pastes having the formulations (% by weight based on net composition, 100 % by weight in total) indicated in Table 13 shown below were prepared. When each tooth paste thus obtained was used, a satisfactory performance was obtained.

**Table 13:**

| Tooth pastes (clear type) | | |
|---|---|---|
| | Example 24 | Example 25 |
| 3-Guanidinopropylmyristylamide pyrrolidone carboxylate | 0.10 | |
| 2-Guanidinoethyldodecanamide | | 0.10 |
| Silicic anhydride | 20.00 | 20.00 |
| Sorbitol solution | 55.00 | 55.00 |
| Glycerin | 10.00 | 10.00 |
| Sodium carboxymethyl cellulose | 1.50 | 1.50 |
| Sodium saccharate | 0.05 | 0.05 |
| Ethyl p-oxybenzoate | 0.01 | 0.01 |
| Dipotassium glycyrrhizinate | 0.01 | 0.01 |
| Cetylpyridinium chloride | 0.10 | 0.10 |
| dl-Tocopherol | 0.02 | 0.02 |
| Fragrance | Appropriate quantity | Appropriate quantity |
| Colorant | Appropriate quantity | Appropriate quantity |
| Purified water | Remainder | Remainder |

### Example 26

The liquid dentifrice having the formulation (% by weight based on net composition, 100 % by weight in total) indicated in Table 14 shown below was prepared. When the liquid dentifrice thus obtained was used, a satisfactory performance was obtained.

**Table 14:**

| Liquid dentifrice | |
|---|---|
| | Example 26 |
| 4-Guanidinobutylpalmitylamide acetate | 0.20 |
| Silicic anhydride | 10.00 |
| Propylene glycol | 2.00 |
| Glycerin | 5.00 |
| Sorbitol solution | 10.00 |
| Sodium polycrylate | 1.00 |
| Sodium lauryl sarcosine | 1.00 |
| Sodium saccharate | 0.10 |
| Ethyl p-oxybenzoate | 0.01 |
| Fragrance | Appropriate amount |
| Colorant | Appropriate amount |
| Purified water | Remainder |

### Example 27

The liquid dentifrice having the formulation (% by weight based on net composition, 100 % by weight in total) indicated in Table 15 shown below were prepared. When the liquid dentifrice thus obtained was used, a satisfactory performance was obtained.

**Table 15:**

| Liquid dentifrice | |
|---|---|
| | Example 27 |
| 2-Guanidinoethyldodecanamide | 0.10 |
| Ethanol | 10.00 |
| Glycerin | 5.00 |
| Sodium lauryl sulfate | 1.00 |
| Polyoxyethylene polyoxypropylene glycol | 0.50 |
| Sodium saccharate | 0.15 |
| Sodium benzoate | 0.01 |
| Fragrance | Appropriate quantity |
| Colorant | Appropriate quantity |
| Purified water | Remainder |

### Examples 28 to 29

The neat mouth washes having the formulations (% by weight based on net composition, 100 % by weight in total) indicated in Table 16 shown below were prepared. When each mouth wash thus obtained was used neat, a satisfactory performance was obtained.

**Table 16:**

| Mouth washes (neat type) | | |
|---|---|---|
| | Example 28 | Example 29 |
| 2-Guanidinoethyldodecanamide | 0.20 | |
| 6-Guandinohexyldodecanamide ascorbate | | 0.20 |
| Ethanol | 15.00 | 15.00 |
| Glycerin | 10.00 | 10.00 |
| Polyoxyethylene hardened castor oil | 2.00 | 2.00 |
| Sodium saccharate | 0.15 | 0.15 |
| Sodium benzoate | 0.05 | 0.05 |
| Sodium dihydrogen phosphate | 0.10 | 0.10 |
| Hinokithiol | 0.10 | 0.10 |
| Fragrance | Appropriate quantity | Appropriate quantity. |
| Colorant | Appropriate quantity, | Appropriate quantity |
| Purified water | Remainder | Remainder |

### Examples 30 and 31

The concent rated mouth washes having the formulations (% by weight based on net composition, 100 % by weight in total) indicated in Table 17 shown below were prepared. When each mouth wash thus obtained was used as being diluted, a satisfactory performance was obtained.

**Table 17:**

| Mouth washes (concentrated type) | | |
|---|---|---|
| | Example 30 | Example 31 |
| 4-Guanidinobutyldodecanamide malate | 0.20 | |
| 4-Guanidinobutylmyristylamide acetate | | 0.20 |
| Isopropyl alcohol | 30.00 | 30.00 |
| Sorbitol solution | 10.00 | 10.00 |
| Sodium lauryl sulfate | 4.00 | 4.00 |
| Sodium benzoate | 0.15 | 0.15 |
| Allantoin | 0.01 | 0.01 |
| Iodine | Appropriate quantity | Appropriate Quantity |
| Fragrance | Appropriate Appropriate quantity | Quantity |
| Colorant | Appropriate quantity | Appropriate Quantity |
| Purified water | Remainder | Remainder |

### Examples 32 and 33

The liquid mouth refreshers having the formulations (% by weight based on net composition, 100 % by weight in total) indicated in Table 18 shown below were prepared. When each mouth refresher thus obtained was used, a satisfactory performance was obtained.

**Table 18:**

| Mouth refreshers (liquid type) | | |
|---|---|---|
| | Example 32 | Example 33 |
| 3-Guanidinopropylmyristylamide hydrochloride | 0.20 | |
| 6-Guanidinohexyldodecanamide acetate | | 0.20 |
| Ethanol | 40.00 | 40.00 |
| Glycerin | 10.00 | 10.00 |
| Polyoxyethylene hardened castor oil | 1.00 | 1.00 |
| 1-Menthol | 0.50 | 0.50 |
| Sodium saccharate | 0.05 | 0.05 |
| Chlorhexidine gluconate | 0.02 | 0.02 |
| Isopropylmethylphenol | 0.01 | 0.01 |
| Fragrance | Appropriate quantity | Appropriate Quantity |
| Purified water | Remainder | Remainder |

### Examples 34 to 37

The mouth washes having the formulations (% by weight based on net composition, 100 % by weight in total) indicated in Table 19 shown below were prepared. When each mouth wash thus obtained was used, a satisfactory performance was obtained.

**Table 19:**

| Mouth waher | | | | |
|---|---|---|---|---|
| | Example 34 | Example 35 | Example 36 | Example 37 |
| 4-Guanidinobutyldodecanamide acetate | 0.20 | | | |
| 4-Guanidinobutyldodecanamide glutamate | | 0.20 | | |
| 2-Guanidinoethyldodecanamide | | | 0.20 | |
| 6-Guanidinohexyldodecanamide hydrochloride | | | | 0.20 |
| Ethanol | 22.00 | 22.00 | 22.00 | 22.00 |
| Eucalyptol | 0.10 | 0.10 | 0.10 | 0.10 |
| Thymol | 0.06 | 0.06 | 0.06 | 0.06 |
| Menthol | 0.04 | 0.04 | 0.04 | 0.04 |
| Benzoic acid | 0.15 | 0.15 | 0.15 | 0.15 |
| Methyl salicylate | 0.06 | 0.06 | 0.06 | 0.06 |
| POLIXAMER 407 | 0.14 | 0.14 | 0.14 | 0.14 |
| Glycerin | 0.50. | 0.50 | 0.50 | 0.50 |
| Propylene glycol | 2.50 | 2.50 | 2.50 | 2.50 |
| Purified water | Remainder | Remainder | Remainder | Remainder |

## Claims

1. An antiseptic or bactericide comprising an amide group-containing guanidine derivative represented by General Formula (I) or a salt thereof: 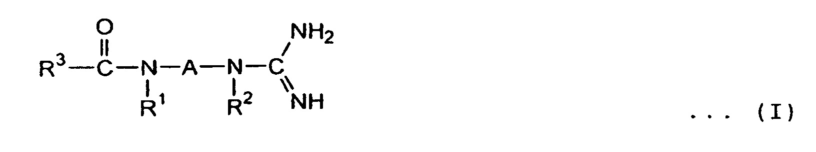 wherein R¹ and R² are the same or different and each denotes a hydrogen atom, a straight or branched alkyl group or alkenyl group having 1 to 4 carbon atoms, R³ denotes a straight or branched alkyl group or alkenyl group having 11 to 13 carbon atoms, and A denotes a straight or branched alkylene group or alkenylene group having 1 to 10 carbon atoms.

2. An antiseptic or bactericide of claim 1 wherein R³-COin said formula (I) is selected from the group consisting of lauroyl group and myristoyl group.

3. An antiseptic or bactericide of claim 1 or claim 2 wherein R¹ in formula (I) is a hydrogen atom.

4. A dermally applicable composition comprising a bactericide or an antiseptic according to any one of claims 1 to 3.

5. A washing composition comprising a bactericide or an antiseptic according to any one of claims 1 to 3.

6. An antibacterial fiber aggregate impregnated with an antiseptic or bactericide according to any one of claims 1 to 3.

7. An antibacterial fiber aggregate whose fiber surface carries an antiseptic or bactericide according to any one of claims 1 to 3.

8. A method for eradicating, or inhibiting proliferation of a microorganism comprising using an amide group-containing guanidine derivative as set out in any one of claims 1 to 3.

## Patentansprüche

1. Antiseptikum oder Bakterizid, umfassend ein amidgruppenhältiges Guanidinderivat der allgemeinen Formel (I) oder ein Salz davon: 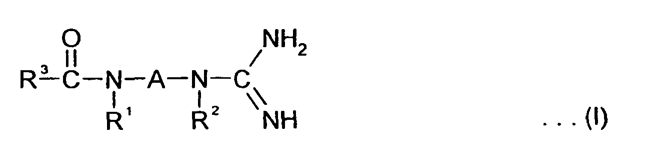 worin R¹ und R² gleich oder unterschiedlich sind und jeweils für ein Wasserstoffatom, eine unverzweigte oder verzweigte Alkylgruppe oder Alkenylgruppe mit 1 bis 4 Kohlenstoffatomen stehen, R³ für eine unverzweigte oder verzweigte Alkylgruppe oder Alkenylgruppe mit 11 bis 13 Kohlenstoffatomen steht und A für eine unverzweigte oder verzweigte Alkylengruppe oder Alkenylengruppe mit 1 bis 10 Kohlenstoffatomen steht.

2. Antiseptikum oder Bakterizid nach Anspruch 1, worin R³-CO- in Formel (I) aus der aus der Lauroylgruppe und der Myristoylgruppe bestehenden Gruppe ausgewählt ist.

3. Antiseptikum oder Bakterizid nach Anspruch 1 oder Anspruch 2, worin R¹ in Formel (I) ein Wasserstoffatom ist.

4. Auf die Haut auftragbare Zusammensetzung, umfassend ein Antiseptikum oder Bakterizid nach einem der Ansprüche 1 bis 3.

5. Waschzusammensetzung, umfassend ein Antiseptikum oder Bakterizid nach einem der Ansprüche 1 bis 3.

6. Antibakterielles Faseraggregat, das mit einem Antiseptikum oder Bakterizid nach einem der Ansprüche 1 bis 3 imprägniert ist.

7. Antibakterielles Faseraggregat, dessen Faseroberfläche ein Antiseptikum oder Bakterizid nach einem der Ansprüche 1 bis 3 trägt.

8. Verfahren zur Vernichtung oder Hemmung der Vermehrung eines Mikroorganismus, umfassend die Verwendung eines amidgruppenhältigen Guanidinderivats nach einem der Ansprüche 1 bis 3.

## Revendications

1. Antiseptique ou bactéricide comprenant un dérivé de guanidine contenant un groupe amide représenté par la formule générale (I) ou un sel de celui-ci : 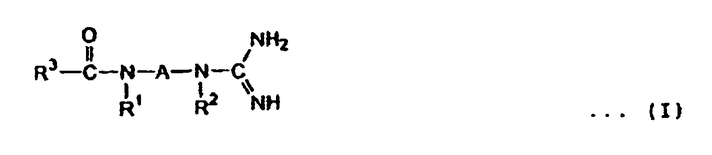 dans laquelle R¹ et R² sont identiques ou différents et désignent chacun un atome d'hydrogène, un groupe alkyle ou un groupe alcényle droit ou ramifié comportant 1 à 4 atomes de carbone, R³ désigne un groupe alkyle ou un groupe alcényle droit ou ramifié comportant 11 à 13 atomes de carbone, et A désigne un groupe alkylène ou un groupe alcénylène droit ou ramifié ayant 1 à 10 atomes de carbone.

2. Antiseptique ou bactéricide selon la revendication 1, dans lequel R³-CO- dans ladite formule (I) est choisi dans le groupe constitué par le groupe lauroyle et le groupe myristoyle.

3. Antiseptique ou bactéricide selon la revendication 1 ou la revendication 2, dans lequel R¹ dans la formule (I) est un atome d'hydrogène.

4. Composition applicable dermiquement comprenant un bactéricide ou un antiseptique selon l'une quelconque des revendications 1 à 3.

5. Composition de lavage comprenant un bactéricide ou un antiseptique selon l'une quelconque des revendications 1 à 3.

6. Agrégat de fibre antibactérien imprégné d'un antiseptique ou d'un bactéricide selon l'une quelconque des revendications 1 à 3.

7. Agrégat de fibre antibactérien dont la surface de fibre porte un antiseptique ou un bactéricide selon l'une quelconque des revendications 1 à 3.

8. Procédé d'éradication ou d'inhibition de la prolifération d'un microorganisme comprenant l'utilisation d'un dérivé de guanidine contenant un groupe amide tel qu'indiqué dans l'une quelconque des revendications 1 à 3.
